# EUROPEAN PATENT SPECIFICATION

(11) **EP 0 804 185 B1**
(45) Date of publication and mention of the grant of the patent: **02.02.2000**
(21) Application number: 93922613.0
(22) Date of filing: 08.09.1993
(51) Int. Cl.: A61K 31/285, A61K 33/36

(54) **ARSENIC MEDICAMENTS FOR THE TREATMENT OF CHRONIC FATIGUE SYNDROME**
ARSEN-ENTHALTENDE ARZNEIMITTEL ZUR BEHANDLUNG DES CHRONISCHEN ERMÜDUNGSSYNDROMS
MEDICAMENTS ARSENICAUX POUR LE TRAITEMENT DU SYNDROME DE FATIGUE CHRONIQUE

(30) Priority: 09.07.1993 IT TO930510
(43) Date of publication of application: 05.11.1997
(73) Proprietor: TARELLO, Walter, I-06061 Castiglione del Lago (Perugia) (IT); SANTAMARIA, Anna, I-06061 Castiglione del Lago (Perugia) (IT)
(72) Inventor: TARELLO, Walter, I-06061 Castiglione del Lago (Perugia) (IT); SANTAMARIA, Anna, I-06061 Castiglione del Lago (Perugia) (IT)
(74) Representative: Marchi, Massimo, Dr.
(86) International application number: IT9300094
(87) International publication number: WO9501789

(56) References cited:
- REV.MED.INTERNE vol. 14, no. 4 , April 1993 pages 233 - 42 'Le syndrome de fatigue chronique: une revue critique'
- AUST.FAM.PHYSICIAN vol. 21, no. 3 , 1992 pages 278 - 285 'Chronic fafigue syndrome. A review from the general practice perspective'

## Description

This invention relates to the use of organic and inorganic compounds of arsenic for the manufacture of a medicament for treating chronic fatigue syndrome.

Chronic fatigue syndrome is a constellation of clinical symptoms including incapacitating exhaustion or fatigue associated with a marked reduction in activity level; in addition to the general fatigue, severe fatigue is typically produced by low levels of exertion, which would have been easily tolerated in the premorbid condition but now result in marked fatigue lasting more than 24 hours.

Other symptoms include malaise, muscle and joint pain, muscle weakness, mild fever, recurrent sore throats, swollen or tender lymph nodes, headaches and a variety of neuropsychological complaints that include dizziness, visual disturbances, irritability, cognitive difficulties like dyslogia, impaired memory, difficulty with word finding, problem solving, and abstract thinking.

Furthermore, chronic fatigue syndrome is characterized by disabling fatigue, as well as neuropsychiatric symptoms including difficulties with concentration, memory and movement.

An effective therapy has not yet been found. Several reports describe abnormalities of muscle metabolism and performance. In some patients profound and transient ataxia of acute onset and transient paresis are concomitant to high levels of creatine phosphokinase (CPK) and deficiences of magnesium. Eosinophilia and monocytosis are often presents.

I have now found that the organic and inorganic compounds of arsenic are very active against the chronic fatigue syndrome.

Arsenic trioxide (As₂O₃) is the reference material for all arsenic compounds. For this reason I selected arsenic trioxide for my personal treatment against chronic fatigue syndrome.

The oral treatment is preferably carried out by administering 2 milligrams of arsenic trioxide (As₂O₃) per 10 kilograms of body weight, every day and for a period of 10-15 days.

The daily dose is preferably divided into three parts. Each part is taken every 8 hours, after or during a meal, and for a period of 10 days, at least. For example, a patient weighting about 70 kilograms will take orally 14 milligrams of arsenic trioxide (As₂O₃), divided into three parts, for a period of 10-15 days.

If needed, one more cycle of treatment can be performed after 40 days.

Arsenic trioxide is not cumulative because it is eliminated by the body in 7-42 days.

Since arsenic organic compounds are less toxic and allow a prompt and quicker restoration of the state of health in the patient, an organic compound containing 20% of arsenic, like sodium arsphenamine (dihydroxy-diammino-arsenobenzene disodium salt), is suggested.

One milliliter of a solution 1:100 of 4,4'-dioxy-3,3'-di amino-arsenobenzene disodium salt contains about 2 milligrams of arsenic.

Intravenous injection of 2 milligrams of arsenic per 10 kilograms of body weight, for 2-4 consecutive days, can resolve all the symptoms of the CFS in few hours or days, depending on how severe and chronic the infection is.

## Claims

1. Use of an organic or inorganic compound of arsenic for the manufacture of a medicament for treating chronic fatigue syndrome.

2. Use of a compound of arsenic according to claim 1, characterized in that the medicament is for oral route.

3. Use of a compound of arsenic according to claim 1 or 2, characterized in that said compound is arsenic trioxide.

4. Use of a compound of arsenic according to claim 1, characterized in that the medicament is for injectable route.

5. Use of a compound of arsenic according to any one of claims from 1 to 4, characterized in that said compound is 4,4'-di oxy-3,3'-diamino-arsenobenzene dihydrochloride.

## Patentansprüche

1. Verwendung einer organischen oder anorganischen Arsenverbindung zur Herstellung eines Medikamentes zur Behandlung des chronischen Müdigkeitssyndroms.

2. Verwendung einer Arsenverbindung nach Anspruch 1, dadurch gekennzeichnet, daß das Medikament für den oralen Weg bestimmt ist.

3. Verwendung einer Arsenverbindung nach Anspruch 1 oder 2, dadurch gekennzeichnet, daß diese Verbindung Arsentrioxid ist.

4. Verwendung einer Arsenverbindung nach Anspruch 1, dadurch gekennzeichnet, daß das Medikament injizierbar ist.

5. Verwendung einer Arsenverbindung nach einem der Ansprüche 1 bis 4, dadurch gekennzeichnet, daß die Verbindung 4,4'-Dioxy-3,3'-diamino-arsenobenzol-dihydrochlorid ist.

## Revendications

1. Utilisation d'un composé organique ou minéral de l'arsenic pour la fabrication d'un médicament pour le traitement du syndrome de fatigue chronique.

2. Utilisation d'un composé de l'arsenic selon la revendication 1, caractérisée en ce que le médicament est destiné à la voie orale.

3. Utilisation d'un composé de l'arsenic selon la revendication 1 ou 2, caractérisée en ce que ledit composé est le trioxyde d'arsenic.

4. Utilisation d'un composé de l'arsenic selon la revendication 1, caractérisée en ce que le médicament est destiné à la voie injectable.

5. Utilisation d'un composé de l'arsenic selon une quelconque des revendications 1 à 4, caractérisée en ce que ledit composé est le dichlorhydrate de 4,4'-dioxy-3,3'-diaminoarsénobenzène.
